(19) Europäisches Patentamt
European Patent Office
Office européen des brevets

(11) Publication number: **0 233 031 B1**

(12) **EUROPEAN PATENT SPECIFICATION**

(45) Date of publication of patent specification: **07.04.93**

(51) Int. Cl.[5]: **C07H 5/02**, C07H 15/14, A61K 31/70, //C07H19/06

(21) Application number: **87300871.8**

(22) Date of filing: **30.01.87**

# (54) Methylthioribose analogues, their preparation and use as medicinal agents and biocides.

(30) Priority: **30.01.86 US 823929**

(43) Date of publication of application:
**19.08.87 Bulletin 87/34**

(45) Publication of the grant of the patent:
**07.04.93 Bulletin 93/14**

(84) Designated Contracting States:
**AT BE CH DE ES FR GB GR IT LI LU NL SE**

(56) References cited:
**US-A- 3 699 103**
**US-A- 4 086 076**

**CABOHYDRATE RESEARCH, vol. 99, no. 1, January 1982, pages 23-31, Elsevier Scientific Publishing Co., Amsterdam, NL; W.L. DILLS, Jr. et al.: "2,5-anhydro-1-deoxy-d-lyxitol, 2,5-anhydro-1-deoxy-d-mannitol, and 2,5-anhydro-1-deoxy-d-talitol. Synthesis and enzymic studies"**

(73) Proprietor: **STATE OF OREGON BY AND THROUGH THE OREGON STATE BOARD OF HIGHER EDUCATION ON BEHALF OF THE OREGON HEALTH SCIENCES UNIVERSITY AND OREGON STATE UNIVERSITY, 3181 S.W. Sam Jackson Park Road
Portland, Oregon 97201(US)**

(72) Inventor: **Risco, Michael K.
19355 S.W. 65th Street
Tualatin Oregon 97202(US)**
Inventor: **Fitchen, John H.
6920 S.E. 28th Avenue
Portland Oregon 97202(US)**
Inventor: **Ferro, Adolph J.
1112 N.W. Charlemagne Corvallis
Oregon 97330(US)**

(74) Representative: **Sheard, Andrew Gregory et al
Kilburn & Strode 30, John Street
London WC1N 2DD (GB)**

EP 0 233 031 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

CHEMICAL ABSTRACTS, vol. 102, no. 3, 21st January 1985, page 20, no. 17155b, Columbus, Ohio, US; L. NOVOTNY et al.: "Structure-intestinal transport and structure-metabolism correlations of some potential cancerostatic pyrimidine nucleosides in isolated rat jejunum", & CANCER CHEMOTHER. PHARMACOL. 1984, 13(3), 195-9; & Eleventh Collective Index, volumes 96-105, 1982-1986, formulas C5H8O2-C8H7T; & Beta-d-ribofuranose,5-chloro-5-deoxy

TETRAHEDRON LETTERS, vol. 26, no. 24, 1985, pages 2885-2888, Pergamon Press Ltd, GB; Y. TAMARU et al.: "Iodoetherification of 4-penten-1,3-diols: Stereoselective synthesis of cis 2-iodomethyl-3-hydroxytetrahydrofurans"

CHEMICAL ABSTRACTS, vol. 101, no. 7, 13th August 1984, page 648, no. 55396t, Columbus, Ohio, US; R. WEN et al.: "Methyleneindolines, indolenines and indoleniniums. XVII. Rearrangement of deethyltabersonine in acetic acid", & HETEROCYCLES 1984, 22(5), 1061-5

TETRAHEDRON LETTERS, vol. 21, 1980, pages 3051-3054, Pergamon Press Ltd, GB; J.-P. BATS et al.: "Transposition des oxirannes-ethanols par l'intermédiaire d'alcoxyetains. Influence de la configuration de l'oxiranne"

TETRAHEDRON LETTERS, vol. 22, no. 45, 1981, pages 4503-4506, Pergamon Press Ltd, GB; S. DAVID et al.: "A case of oligomerization on attempted chlorination of an alpha-hydroxy acetal with the triphenylphosphine tetrachloromethane reagent"

CHEMICAL ABSTRACTS, vol. 95, no. 21, 23rd November 1981, page 214, no. 182436p, Columbus, Ohio, US; S.K. SHAPIRO et al.: "5-methylthioribose as a precursor of the carbon chain of methionine", & BIOCHEM. BIOPHYS. RES. COMMUN. 1981, 102(1), 302-7

CHEMICAL ABSTRACTS, vol. 80, no. 11, 18th March 1974, page 151, no. 57296g, Columbus, Ohio, US; H.T. SCHROEDER et al.: "Biological production of 5-methylthioribose.", & CAN. J. MICROBIOL. 1973, 19(11), 1347-54

Kushad et al, Plant Physiology, 1985, 79(2), 525-9, discloses ethylthioribose (ETR), isopropylthioribose (iPTR) and isobutylthioribose (iBTR) as inhibitors of MTR-kinase present in tomatoes

Methods in enzymology, 1983, 94, 361-364, discloses that ETR, PTR, i-PTR, BTR and iBTR are substrates of MTR-kinase isolated from E.aerogenes

Biochem. Pharmacol., 1980, 29(13), 1963-5, discloses that iBTR is a metabolite of isobutylthioadenosine

Biochimica et Biophysica Acta, 1976, 438, 487-494, discloses PTR and ETR as products of an MTA nucleosidase

J. Med. Chem, 1974, 17(11), 1197-207, discloses ETR as an intermediate

## Description

The present invention relates to novel methylthioribose analogues, a process for their preparation and their use as medicinal and biocidal agents.

5-Deoxy-5-methylthioribose (MTR) is a naturally occurring compound derived from 5'-deoxy-5'-methylthioadenosine (MTA). MTR is essential in the salvage of methionine in organisms containing the enzyme MTR kinase. MTR is a substrate for MTR kinase, and its carbohydrate and alkylthio moeities are recycled into methionine. Methionine, an essential amino acid, is required for DNA/RNA/protein synthesis.

U.S. 4,420,489 discloses 5-thio-D-ribose and 5-thio-2-Deoxyribose (compounds in which the ring oxygen atom is replaced by a sulfur atom) as agents capable of preventing radiation damage.

U.S. 3,836,644; 3,767,800 and 4,481,196 disclose glycoside phosphate derivatives, some of which could be regarded in the broadest sense as analogs of MTR. In each case, the compounds demonstrate some physiological activity, but not biocidal activity.

U.S. 4,378,369 discloses that some mono- and diesters of 2,5-anhydro-D-mannitol are useful in treating diabetes. These compounds are similar, but not identical to MTR.

U.S. 4,243,663 discloses an adjuvant compound based on fructose instead of ribose.

U.S. 4,086,076 discloses tetrahydrofuranyl based compounds with fungicide and bacteriacide properties.

U.S. 2,840,587 discloses the synthesis of ethionine.

U.S. 4,080,465 discloses cyclic thio compounds.

Shapiro et al., Biochem. Biophys. Res. Comm., 102 302 (1981), discloses that MTR is the precursor of methionine in procaryotic microorganisms.

Schroeder et al., Can. J. Microbiol., 19, 1347 (1973), discloses the production of MTR in E. coli. The article further indicates that MTA is the precursor of MTR in procaryots.

Kikugawa et al., Tetrahedron Letters 2, 87-90 (1971), describes the synthesis of 5'-chloro-5'- deoxyadenosine and similar compounds which serve as synthetic starting points for ethylthioadenosine (ETA). Biochem. Biophys. Acta., 320 357-362 (1973), describes the preparation of MTR.

Plant Physiology 71; 932 (1983) and Eur. J. Biochem. 87; 257 (1978) disclose isobutyl thioribose.

Eur. Biochem., 87, 257 (1978) discloses 5-S-(2-methylpropyl)-5-thio-D-ribose.

Chem. Lett. 819 (1979) discloses orthophenylmethyl thioribose.

Agents for systemically treating fungal infections are known. Amphoterecin-B is frequently prescribed; however, it can cause undesirable side effects, e.g., renal failure.

Kushad et al, Plant Physiology, 1985, 79(2), 525-9, discloses ethylthioribose (ETR), isopropylthioribose (iPTR) and isobutylthioribose (iBTR) as inhibitors of MTR-kinase present in tomatoes.

Methods in enzymology, 1983, 94, 361-364, discloses that ETR, PTR, i-PTR, BTR and iBTR are substrates of MTR-kinase isolated from E.aerogenes.

Biochem. Pharmacol., 1980, 29(13), 1963-5, discloses that iBTR is a metabolite of isobutylthioadenosine.

Biochimica et Biophysica Acta, 1976, 438, 487-494, discloses PTR and ETR as products of an MTA nucleosidase.

J. Med. Chem, 1974, 17(11), 1197-207, discloses ETR as an intermediate.

It is an object of this invention to provide novel methylthioribose analogs which have improved properties, e.g., those discussed above.

It is a further object of this invention to provide the use of a methylthioribose analogue in the manufacture of a medicament for treating mammals infected with a microorganism.

Yet another object of this invention is to provide a method of inhibiting the growth of infectious microorganisms with a methylthioribose analogue.

Upon further study of the specification and appended claims, further objects and advantages of this invention will become apparent to those skilled in the art.

In a first aspect, this invention relates to compounds of Formula Ia

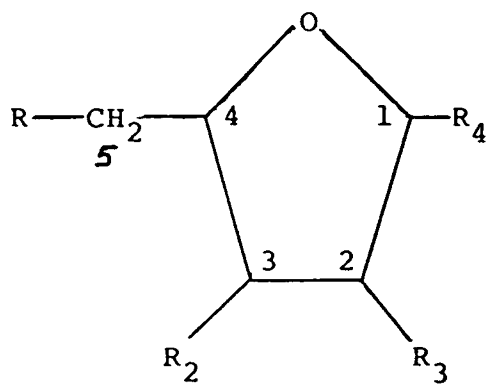

(Ia)

wherein R is $R_1S$- in which $R_1$ is $C_1$-$C_{10}$ linear or branched chain alkyl or halogenated $C_1$-$C_{10}$ linear or branched chain alkyl,

wherein $R_2$, $R_3$ and $R_4$ are the same or different and each is H or -OH,

with the proviso that at least one of $R_2$, $R_3$ and $R_4$ is hydroxy and the further proviso that when $R_2$, $R_3$ and $R_4$ are all OH, $R_1$ is halogenated linear or branched chain alkyl.

In a second aspect, this invention relates to a pharmaceutical or veterinary composition, for example in unit dosage form, comprising, in admixture with a pharmaceutically or veterinarily acceptable carrier, an amount per unit dosage effective to inhibit the growth, for example, of Candida albicans of a compound of Formula Ib

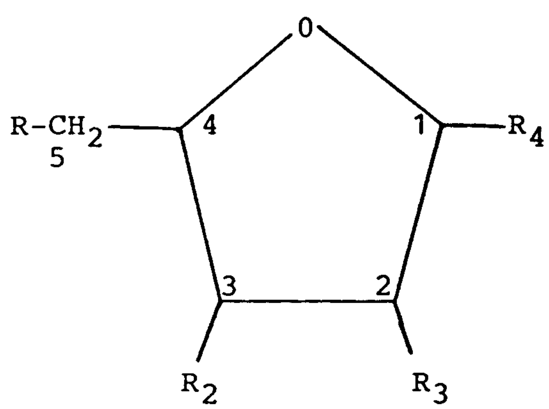

(Ib)

wherein R is H, Cl, F, Br, I or $R_1S$- in which $R_1$ is $C_1$-$C_{10}$ linear or branched chain alkyl or halogenated $C_1$-$C_{10}$ linear or branched chain alkyl,

wherein $R_2$, $R_3$ and $R_4$ are the same or different and each is H or -OH,

with the proviso that at least one of $R_2$, $R_3$ and $R_4$ is H- or -OH and the further proviso that when $R_2$, $R_3$ and $R_4$ are all -OH, $R_1$ is other than methyl.

In a third aspect, the invention relates to a compound of formula Ib, as defined above, for use in human or veterinary medicine, particularly in the treatment of microorganism infections in mammals.

In a fourth aspect, this invention relates to a method of inhibiting the growth of an MTR kinase-containing microorganism, comprising applying to the habitat thereof a metabolization-inhibiting amount of a compound of Formula Ib, as defined above.

In a fifth aspect, this invention relates to the use of a compound of formula Ib, as defined above, in the preparation of an antimicrobial, for example antifungal or antiprotozoal, agent. ("Antimicrobial" is used in the sense of "microbe" meaning "microorganism" and not merely "visible bacterium".)

In a sixth aspect, the invention relates to a process for preparing a compound of formula Ia, as defined above, which comprises hydrolysing a compound of formula IIa

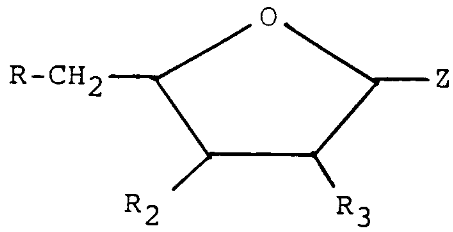

IIa

wherein R, $R_2$ and $R_3$ are as defined for formula Ia, and Z is a hydrolysable group.

In a seventh aspect, the invention relates to a process for preparing a pharmaceutical or veterinary composition, the process comprising admixing a compound of formula Ib with a pharmaceutically or veterinarily acceptable carrier.

This invention is based on the discovery that analogues of MTR inhibit the growth of a wide variety of pathogenic microorganisms. A class of microorganisms inhibited by MTR analogues are MTR kinase-containing microorganisms. MTR kinase is present in many microorganisms, e.g., fungi, bacteria and some types of plants, e.g., tomatoes, apples and avocados.

MTR may be synthesized from MTA (5'-Doeoxy-5'-methylthioadenosine) via MTA nucleosidase and is a substrate for MTR kinase. Neither of these enyzmes is found in mammal cells. The product of the kinase activity, MTR-1-phosphate, is recycled into methionine, which is essential for DNA/RNA and protein synthesis.

It is believed that some of the compounds of this invention manifest their activity by inhibiting the production of methionine and that other compounds of the invention may also function as a substrate for MTR kinase and produce toxic metabolic analogs of methionine, and other analogues possibly function to inhibit growth in a manner distinct from MTR kinase and the methionine recycling pathway. For example, ethionine is known to be toxic (see "Molecular Aspects of the In Vivo and In Vitro Effects of Ethionine, an Analog of Methionine," Microbiological Reviews, Vol. 46, 283-295). Trifluoromethionine is also toxic.

Examples of $R_1$ alkyl groups are ethyl, propyl, isopropyl, butyl, isobutyl, tertbutyl, pentyl, neopentyl, hexyl, heptyl, decyl, etc. The $R_1$ alkyl groups can optionally be mono- to poly- substituted by halogen atoms, e.g., $R_1$ can be chloromethyl, chloroethyl, dichloromethyl, 1,1-dichloroethyl, 1,2-dichloroethyl, bromomethyl, iodomethyl, etc.

Novel compounds of Formula Ia are those where R is H, Cl, F, Br, I or $R_1S$ in which $R_1$ is $C_1$-$C_{10}$ linear or branched chain alkyl or halogenated $C_1$-$C_{10}$ linear or branched chain alkyl, wherein $R_2$, $R_3$ and $R_4$ are the same or different and each is H- or -OH. However, at least one of $R_2$, $R_3$ and $R_4$ is hydroxy and when $R_2$, $R_3$ and $R_4$ are all OH, $R_1$ is other than methyl, or isobutyl (which latter compounds are known in the prior art).

The compounds of Formula Ib can be prepared by conventionally reacting a conventional compound of Formula II

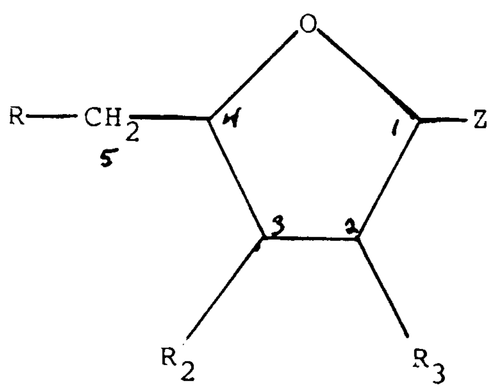

II

wherein $R_2$ and $R_3$ are the same or different and each is H or -OH and Z is hydrolyzable by cleavage in acid solution, (e.g., Z is adenine, guanine, cytosine, uracil, thymine, etc.). The 5' position of the nucleoside is susceptible to suitable halogenating agents, e.g., thionyl chloride or thionyl bromide. The resultant 5'-halogenated-nucleoside can be directly hydrolyzed to the corresponding 5-halogenated compounds of Formula Ib. Alternatively, the 5'-halogenated nucleoside of Formula II can be converted to the

corresponding 5'- alkylthiol derivative by reaction with an alkylthiol, e.g., methylthiol, ethylthiol, etc., in liquid $NH_3$ in the presence of sodium metal. In general, the reaction is carried out under conditions wherein one ml of liquid $NH_3$ is employed for each mmole of nucleoside to be added and, in general, a 3-fold excess of alkyl mercaptan is employed. In general, the reaction proceeds for 1-2 hours. The aqueous fraction is preferably concentrated in vacuo from which the resultant crystals may be collected. Preferably, the resultant crystals are purified by thin layer chromotography. The purified alkylthiol-nucleosides are then hydrolyzed to the appropriate alkylthiol-containing compound by simple acid-catalyzed hydrolysis, e.g., by suspending the nucleoside in a solution of HCl at 100° C. After the reaction has progressed substantially to completion the reaction mixture is cooled and the pH is raised, e.g., to 3.5. The desired product can be separated from the other hydrolysis products, e.g., adenine and any residual unhydrolyzed nucleoside, by cation exchange chromotography.

In general, the preparation of the compounds of Formula Ib is fully conventional and employs conditions well known to those in the art, perhaps requiring only a few routine orientation experiments to determine optimal parameters. The compounds of Formula II are all well known or can be conventionally prepared from known starting materials. The preparation of compounds of Formula Ib may proceed with or without racemization.

The compounds of Formula Ib are valuable biocides and useful as pharmaceuticals. In general, the compounds of Formula 1b have no apparent effect on the growth and differentiation of non-MTR kinase-dependent microorganisms. Thus, the compounds can be used in the treatment of mammals infected with an MTR kinase-dependent microorganism to ameliorate the infection without adversely affecting the infected host. Additionally, the compounds of Formula 1b can be employed to kill or inhibit the growth of MTR kinase-dependent microorganisms by applying them to the habitat of such microorganisms. Often such a habitat is mammalian skin. Thus, compounds of Formula 1b are also suitable for topical application. The compounds of Formula 1b are acid-stable and stable at elevated temperatures Thus, they are especially suitable for oral administration.

In general, the compounds of Formula 1b are effective to inhibit the growth of any MTR kinase-containing microorganism.

The following fungi are illustrative of those whose growth is inhibited by the MTR analogues of this invention:

Candida albicans, Cryptococcus neoformans, Blastomyces dermatitidis, Histoplasma casulatum, Coccidioides immitis, Para coccidioides brasiliensis.

Examples of bacteria whose growth is inhibited by the MTR analogues of this invention are: Enterobacter aerogenes, Staphylococcus aures.

The above lists are not all-inclusive.

MTR kinase-containing microorganisms can be identified by the following method: the assay mixture contains 50 mMolar imidazole-HCl (pH 7.3), 0.1 mMolar [methyl-$C^{14}$] methylthioribose ($5x10^6$ CPM/uM0, 1.0 mMolar ATP, 5.0 mMolar $MgSO_4$, 10 mMolar dithiothrietal and cell extract in a total volume of 0.2 ml. After incubation at 37° C for 20 minutes, the reaction is stopped by addition of 3 volumes of ethanol and the precipitated protein is removed by centrifugation. A 0.2 ml aliquot of the supernatant fluid is then applied to a DOWEX-I-X8 formate column (0.7 x 2 cm) and washed with 0.01 N sodium formate. The product of the reaction, MTR-I-phosphate, is eluted with 12 ml 0.75 N sodium formate. 3 ml of the eluant is combined with scintillation fluid and the amount of product formed is determined by scintillation counting. A reading higher than background indicates a positive test.

Formula 1b is not all-inclusive of compounds effective to inhibit the growth of MTR kinase-containing microorganisms. Thus, any compound which is a substrate for MTR kinase or competes with binding of MTR kinase to MTR can be employed to inhibit the growth of MTR kinase-containing microorganisms or to treat a mammal infected with an MTR kinase-containing microorganism to ameliorate the infection. The use of such compounds is a contemplated equivalent of this invention.

In general, compounds of this invention can be used to treat any infection of a mammal with an MTR kinase-containing microorganism. Specific examples of diseases which can be treated include: Candidiasis; skin lesions, systemic bacteremia, e.g., oral Candidiasis (thrush), Vulvovaginal Candidiasis, Intertridinous Candidiasis, Branchopulmonary Candidiasis, Cryptococcal meningititis, North American blastomycosis, Histoplasmosis, Coccidioidomycosis, Paracoccidioidomycosis.

The compounds of this invention can be employed to treat, mammals, e.g., humans, infected with protozoans, e.g., parasitic protozoans, e.g., Plasmodium falciparum (responsible for malaria), Giardia lamblia (responsible for "hiker's sickness") and Ochromonas malhamensis.

The pharmacologically active compounds of this invention can be processed in accordance with conventional methods of galenic pharmacy to produce products for administration to patients, e.g.,

mammals including humans. Conventional excipients are pharmaceutically acceptable organic or inorganic carrier substances suitable for parenteral or enteral or topical application which do not deleteriously react with the active compounds. Suitable pharmaceutically acceptable carriers include but are not limited to water, salt solutions, alcohols, gum arabic, vegetable oils, polyethylene glycols, gelatin, lactose, amylose, magnesium stearate, talc, silicic acid, viscous paraffin, perfume oil, fatty acid monoglycerides and diglycerides, pentaerythritol fatty acid esters, hydroxymethylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

For parenteral application, particularly suitable are injectable sterile solutions, preferably oil or aqueous solutions, as well as suspensions, emulsions, or implants, including suppositories. Ampoules are convenient unit dosages.

For enteral administration, particularly suitable are tablets, dragees, suppositories or capsules having talc and/or a carbohydrate carrier or binder or the like, the carrier preferably being lactose and/or corn starch and/or potato starch. A syrup, elixir or the like can be used wherein a sweetened vehicle is employed. Sustained release compositions can be formulated, including those wherein the active compound is protected with differentially degradable coatings, e.g., by microencapsulation, multi coatings, etc.

Generally the compounds of this invention are dispensed in unit dosage form comprising 150-1,500 mg in a pharmaceutically acceptable carrier unit dosage.

The dosage of the compounds according to this invention generally is 5 - 30 mg/kg/day when adminstered to patients, e.g., humans, as an anti-fungal or anti-bacterial agent. Amphotericin-B is the currently prescribed known anti-fungal agent and the compounds of this invention can be administered similarly thereto.

Dosages for a given host can be determined using conventional considerations, e.g., by customary comparison of the differential activities of the subject compound and of a known agent, e.g., by means of an appropriate, conventional pharmacological protocol.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the preceding text and the following examples, all temperatures are set forth uncorrected in degrees Celsius and all parts and percentages are by weight, unless otherwise indicated.

Example 1: 5'-chloro-5'-deoxyadenosine

A solution of 0.75ml of thionyl chloride and hexamethylphosphoramide is stirred under nitrogen while 0.5 gms of the nucleoside is added. The mixture is allowed to stir for 15-20 hours at room temperature, in ca. 20 mls of water. The pH is then neutralized to 7-8 with 2N $NH_4OH$. After cooling, the crystals are collected by filtration. The crystals are then applied to DOWEX-50$H^+$, the resin washed well with water and then with 2N $NH_4OH$ to remove the product. After concentration, the second crop of crystals is collected by filtration. The crystals are 5'-chloro- 5'-deoxyadenosine.

Example 2: 5'-chloro-2', 5-deoxyadenosine

Analogously to Example 1, 2'-deoxyadenosine is converted to the captioned product.

Example 3: 5'-chloro-3',5'-deoxyadenosine

Analogously to Example 1, 3'-deoxyadenosine is converted to the captioned product.

Example 4: 5'-chloro-2',3'-deoxyadenosine

Analogously to Example 1, 2',3'-deoxyadenosine is converted to the captioned product.

Example 5: 5'-Chloroarabinoadenosine

Analogously to Example 1, arabinoadenosine is converted to the captioned product.

Example 6: 5'bromo-5'-deoxynucleosides

Analogously to Examples 1-5, the corresponding nucleosides therein are converted to the corresponding 5'-bromo-5'-deoxynucleosides by substitution of thionyl bromide for the thionyl chloride employed therein.

PREPARATION OF HALOGENATED DEOXYRIBOSES

Example 7: 5-deoxy-5-chlororibose (5-chlororibose)

The 5'-chloro-5'deoxy-adenosine of Example 1 is hydrolyzed to 5-chlororibose as follows: 20 mg of the nucleoside is suspended in 4 ml of 0.1 M/HCl at 100 ° C. After 2.5-3 hours, the reaction mixture is cooled and the pH is raised to 3.5. Separation of 5-Chlororibose from adenine (the other hydrolysis product) and any residual unhydrolyzed nucleoside is accomplished through cation exchange chromotography using DOWEX-50$H^+$. The measurement of the sugar concentration is determined using ribose as a standard in the phloroglucinol procedure detailed in Ashwell, G., (1972) Methods Enzymol 8, 85-95.

Example 8: 5-chloro-2,5-deoxyribose

Analogously to Example 7, the product of Example 2 is converted to the captioned product.

Example 9: 5-chloro-3,5-deoxyribose

Analogously to Example 7, the product of Example 3 is converted to the captioned product.

Example 10: 5-chloro-2,3-deoxyribose

Analogously to Example 7, the product of Example 4 is converted to the captioned product.

Example 11: 5-chloro-arabinose

Analogously to Example 7, the product of Example 5 is converted to the captioned product

Example 12

The corresponding 5-bromoriboses are prepared from the products of Example 6 analogously to the procedure in Example 7.

PREPARATION OF 5'-ALKYLTHIO-5'-DEOXYNUCLEOSIDES

The halogenated nucleosides of Examples 1-6 can be converted into the corresponding alkylthiodeoxy nucleosides by reacting the products of Examples 1-6 with the appropriate alkylthiol, i.e., alkylmercaptan, to produce the desired alkylthiodeoxynucleoside. Accordingly, the following examples are merely illustrative.

Example 13: 5'-ethylthio-5'deoxyadenosine

Liquid $NH_3$ is collected in a heavy glass tube immersed in a dry ice-ethanol bath. 1 ml of $NH_3$ is employed for each mole of 5'-chloro-5'-deoxyadenosine. A three-fold excess of ethanolthiol and a small amount of sodium methal is added. Dry 5'-chloro-5'-deoxyadenosine is added and the reaction proceeds for 1-2 hours. After the reaction is complete, the $NH_3$ is evaporated under vacuum and the residual mercaptan is removed by three successive ether extractions. The aqueous fraction is subsequently concentrated in vacuo, the resultant crystals are collected and the nucleoside purified by a thin layer of chromatography.

Example 14: 5'-propylthio-5'-deoxyadenosine

Analogously to Example 13, the product of Example 1 is reacted with propanethiol to produce the captioned product.

## HYDROLYSIS OF THE NUCLEOSIDES

### Example 15: 5-ethylthio-5-deoxyribose (ethylthioribose) (ETR)

The product of Example 13 is suspended in 4 ml of 0.1m HCl at 100° C. After 2.5-3 hours, the reaction mixture is cooled and the pH is raised to 3.5. Separation of the desired product from adenine and any residual unhydrolyzed nucleoside is accomplished through cation exchange chromotography using DOWEX $50H^+$.

### Example 16: 5-propyl-5-deoxyribose

Analogously to Example 15, the product of Example 14 is hydrolyzed to produce the captioned product.

## INHIBITION OF THE GROWTH OF MTR KINASE-DEPENDENT MICROORGANISMS

In the following examples, an MTR kinase-dependent microorganism is treated in vitro with compounds of this invention. In the following examples, LB is a growth medium (Luria-Bertani) containing, per liter, 10 grams Bacto-tryptone, 5 grams Bacto-yeast extract and 10 grams NaCl. $OD_{550}$ is a measure of the optical density of the suspension at the indicated time. Low optical density indicates a low cell concentration in the suspension and thus that compounds were effective to inhibit the growth of the microorganisms. An optical density of zero indicates that no growth occurred.

Example 17: Treatment of Candida albicans with 5-Deoxy-5-Ethylioribose

| Tube # | Additions ETR Stock | [ ] | LB broth ML | 0.1ml Cells | $OD_{550}$ at 24 Hrs | Growth at 7 days |
|---|---|---|---|---|---|---|
| 1 | 1 ml* | 9.2mM* | 1.0 | $10^4$ | 0.0 | -- |
| 2 | 0.5 | 4.6mM | 1.50 | $10^4$ | 0.0 | -- |
| 3 | 0.25 | 2.3mM | 1.75 | $10^4$ | 0.0 | -- |
| 4 | 0.1 | 0.9mM | 1.90 | $10^4$ | 0.120 | + |
| 5 | 0.05 | 0.46mM | 1.95 | $10^4$ | 0.357 | ++ |
| 6 | 0.025* | 0.23mM | 1.975 | $10^4$ | 0.355 | ++ |
| 7 | 0.010 | 0.092mM | 1.99 | $10^4$ | 0.409 | ++ |
| 8 | Control | | 2.0 | $10^4$ | 0.413 | ++ |

*Total Vol. = 2.0 mls.

Example 18: Treatment of Candida albicans with 5-Deoxy-5-Ethylthioribose (ETR)

(ETR* vs High Dose Candida inoculum)

| Tube # | LB broth | ETR | $H_2O$ | Blasto-candida | 24 hrs $OD_{550}$ | Growth at 72 Hrs |
|---|---|---|---|---|---|---|
| 1 | 1.5mls | 0.5ml | | $10^2$ | 0.000 | -- |
| 2 | 1.5mls | 0.5ml | | $10^3$ | 0 | -- |
| 3 | 1.5mls | 0.5ml | | $10^4$ | 0 | -- |
| 4 | 1.5mls | 0.5ml | | $10^5$ | 0 | -- |
| 5 | 1.5mls | 0.5ml | | $10^6$ | 0.006 | -- |
| 6 | 1.5mls | 0.5ml | | $10^7$ | 0.008 | -- |
| 7 | 1.5mls | -- | 0.5mls | $10^2$ | 0.488 | ++ |
| 8 | 1.5mls | -- | 0.5mls | $10^3$ | 0.496 | ++ |
| 9 | 1.5mls | -- | 0.5mls | $10^4$ | 0.574 | ++ |
| 10 | 1.5mls | -- | 0.5mls | $10^5$ | 0.606 | ++ |
| 11 | 1.5mls | -- | 0.5mls | $10^6$ | 0.658 | ++ |
| 12 | 1.5mls | -- | 0.5mls | $10^7$ | 0.716 | ++ |

*Using 18.4 mM stock solution of ETR.

Examples 17 and 18 indicate that 5-Deoxy-5-ethyl-5-deoxy-5-ethylthioribose clearly inhibits the growth of Candida albicans.

Example 19

In this example, samples containing a known number of microorganisms were colonized without ETR and in the presence of ETR. In the instances where growth occurred, one colony formed for each microorganism. The microorganisms colonized in the presence of ETR formed less than 1,000 colonies, indicating that ETR is cytocidal to Candida albicans, as opposed to cytostatic.

Procedure:

| | Stock Candida | Additions LB Broth | ETR | Time | Amount plated | | Colonies |
|---|---|---|---|---|---|---|---|
| 1. | -- | 0.1 ml | -- | 0 hr | 0.1 | 1 | 0 |
| 2. | (0.1ml)$10^3$ orgs | 1.9 ml | -- | 0 hr | 50 | 1 | 26 |
| 3. | (0.1ml)$10^3$ orgs | 1.9 ml | -- | 0 hr | 100 | 1 | 57 |
| 4. | (0.1ml)$10^3$ orgs | 1.9 ml | -- | 3 hrs | 100 | 1* | 116 |
| 5. | (0.1ml)$10^3$ orgs | 1.9 ml | 0.5ml | 3 hrs | 100 | 1* | 2 |
| 6. | (0.1ml)$10^3$ orgs | 1.9 ml | -- | 5 hrs | 100 | 1* | 327 |
| 7. | (0.1ml)$10^3$ orgs | 1.9 ml | 1.5ml | 5 hrs | 100 | 1* | 0 |

*1:10 dilution

Interpretation of Results:

| Vessel | | Colonies | Dilution Factor | Total # Organisms |
|---|---|---|---|---|
| 1. | BLANK | 0 | 1 | 0 |
| 2. | Control (no ETR) | 26 | $\frac{50\ 1}{2000\ 1}$ or 40 | 1040 |
| 3. | Control (no ETR) | 57 | $\frac{100\ 1}{2000\ 1}$ or 20 | 1120 |
| 4. | 3 hrs incubation | 116 | $\frac{100\ 1}{2000\ 1}$ *or 200 | 23200 |
| 5. | 3 hrs (+ETR) 4mM | 2 | $\frac{100\ 1}{2000\ 1}$ *or 200 | 400 |
| 6. | 5 hrs (w/o ETR) | 327 | $\frac{100\ 1}{2000\ 1}$ *or 200 | 65400 |
| 7. | 5 hrs (w/ETR) 4mM | 0 | $\frac{100\ 1}{2000\ 1}$ *or 200 | 0 |

* 1:10 dilution

Example 20: Treatment of Candida albicans with 5-Chloro-5-Deoxyribose

| Tube # | 10mM Stock 5-chloro | LB | Cells | $OD_{550}$ 24 hrs | $OD_{550}$ 7 days |
|---|---|---|---|---|---|
| 1 | -- | 2.0ml | $10^4$ (.1ml) | 0.431 | 1.739 |
| 2 | 0..2 | 1.8 | $10^4$ | 0.0 | 0.0 |
| 3 | 0.4 | 1.6 | $10^4$ | 0.0 | 0.0 |
| 4 | 0.8 | 1.2 | $10^4$ | 0.0 | 0.0 |

Example 20 demonstrates that 5-chlororibose is effective to kill Candida albicans.

TREATMENT OF BONE MARROW CELLS WITH ETHYLTHIORIBOSE

In the following examples, human bone marrow and mouse marrow cells were subjected to ethylthioribose. In each case, a preparation of immature blood cells from bone marrow were conventionally stimulated to mature in a gel matrix. Immature human granulocytic precursor (CFU-GM) cells formed a colony in the matrix and the number of cells in the colony was counted. Such tests are believed to be a good measure of the toxicity of the subject compounds to humans.

Example 21

ETR: Toxicity toward Human Bone Marrow Function in vitro CFU-GM assays as usual
30cc at $8.9 \times 10^6$/cc, LD $10.4 \times 10^6$/cc (5cc)
Total volume of each culture - 1 ml.

| | Marrow | ETR | | P+ Serum (not applicable) | C' (not applicable) | CFU-GM($2 \times 10^5$ total cells/plate |
|---|---|---|---|---|---|---|
| 1 | + | -- | | " | " | 54,57 |
| 2 | + | 100ml | (stock)* | | | 50,51 |
| 3 | + | 100" | (1:1) | | | 51,52 |
| 4 | + | 100" | (1:2) | | | 50,53 |
| 5 | + | 100" | (1:4) | | | 54,55 |
| 6 | + | 100" | (1:10) | | | 53,51 |
| 7 | + | 100" | (1:20) | | | 55,52 |
| 8 | + | 100" | (1:40) | | | 54,55 |
| 9 | + | 100" | (1:50) | | | 52,56 |
| 10 | + | 100" | (1:100) | | | 53,53 |
| 11 | + | 100" | (1:1000) | | | 54,51 |
| 12 | + | 100" | (1:10k) | | | 51,50 |
| 13 | + | 100" | (1:100k) | | | 55,53 |

*Concentration of ETR Stock - 18.4 mM

Example 22

ETR: Toxicity toward mouse marrow

| | ($0x10^{6}$/ml Marrow | ETR | CFU-GM |
|---|---|---|---|
| 1 | 10 | none | 37,40 |
| 2 | 10 | 100 (stock) | 35,36 |
| 3 | 10 | 100 (1:1) | 34 -- |
| 4 | 10 | 100 (1:2) | 37,33 |
| 5 | 10 | 100 (1:4) | 32,34 |
| 6 | 10 | 100 (1:10) | 36,34 |
| 7 | 10 | 100 (1:40) | 33,35 |
| 8 | 10 | 100 (1:50) | 37,36 |
| 9 | 10 | 100 (1:100) | 39 -- |
| 10 | 10 | 100 (1:1k) | 36,37 |
| 11 | 10 | 100 (1:10k) | 35,32 |
| 12 | 10 | 100 (1:100k) | 33,35 |
| 13 | 10 | 100 (1:20k) | 32,34 |

The following examples illustrate the effect of ETR on the growth of protozoans.

Example 23: Toxicity of ETR to Ochromonas malhamensis (ATCC #11532)

a. Procedure. 2 ml cultures of O. malhamensis were set up at a cell density of $4x10^{4}$ per ml. The culture medium used was thioglycolate broth and incubation took place at ambient temperature near a south-facing window (these organisms are photosynthetic). ETR was added at the appropriate dilution at the beginning of the experiment. Cell number was determined using a hemacytometer. The data (shown below) were obtained after 48 hours of incubation.

b. Results.

| Condition | Cell density (Day 2) |
|---|---|
| Control (no additions) | 1.28 x $10^{6}$ per ml |
| + $10^{-5}$M ETR | 1.25 x $10^{6}$ per ml |
| + 2 x $10^{-5}$M ETR | 0.6 x $10^{6}$ per ml |
| + 5 x 10-5M ETR | 0.03 x $10^{6}$ per ml |
| +10 x $10^{-5}$M ETR | 0.03 x $10^{6}$ per ml |
| +20 x $10^{-5}$M ETR | debris |

The results indicate that the $ID_{50}$ for ETR vs. O. malhamensis is 20μM.

Example 24: Inhibition of Giardia lamblia (Portland 1 strain) by ETR

a. Procedure. Giardia lamblia was cultured in Diamond's TP-S-1 medium supplemented with 10% heat-inactivated calf serum and antibiotics (penicillin and streptomycin). The antibiotics were added to prevent contamination by bacteria. Tube cultures containing 7 mls of medium were seeded with 5 x $10^{4}$ organisms per ml and incubated horizontally at 37° C for 48 hrs. ETR was added at the beginning of the experiment. Cell number was determined with the aid of a hemacytometer.

b. Results.

| Condition | Total Cell Number (x $10^{-5}$) 5/23/86* | 11/7/86 | 11/22/86 | Average(SD) |
|---|---|---|---|---|
| Control | 7.65 | 6.0 | 8.2 | 7.28 (0.9) |
| +ETR (0.078 mM) | - | 6.0 | - | 6.0 |
| +ETR (0.156 mM) | 7.4 | 5.0 | 7.0 | 6.5 (1.0) |
| +ETR (0.312 mM) | 6.4 | 2.1 | 4.8 | 4.4 (1.7) |
| +ETR (0.625 mM) | 4.3 | 1.0 | 3.7 | 3.0 (1.4) |
| +ETR (1.24 mM) | 4.1 | 0.6 | 2.8 | 2.5 (1.4) |
| +ETR (2.50 mM) | 2.1 | debris | 1.5 | 1.8 (0.3) |
| +ETR (5.0 mM) | 0.3 | debris | 0.7 | 0.5 (0.2) |

*Cysteine added to the point of precipitation.

The results indicate that the $ID_{50}$ for ETR vs. G. lamblia appears to be near 0.45mM. There is significant cell death and lack of "swimming" at concentrations ranging from 0.62mM to 5mM.

Example 25. Effect of ETR on Plasmodium falciparum in vitro

a. Procedure. Parasitized erythrocytes from a stock four-day-old culture at approximately 10% infection were mixed with washed clean erythrocytes to give a final parasitemia of approximately 1%. Using 1.2 cm plastic Petri dishes duplicate 1 ml cultures were initiated with drug dilutions in complete RPMI-1640, containing 0.1 ml of erythrocytes and 50 ug of gentamycin/ml. The cultures were supplemented with RPMI-1640 (25 mM HEPES), 0.24% sodium bicarbonate and 10% human serum (Type $A^-$). All cultures were gassed with 90% nitrogen, 5% carbon dioxide and 5% oxygen prior to incubation at 37° C. Parasite growth was assessed by microscopic evaluation of Giemsa-stained thin-smears.

b. Results.

| Conditions | Percent Parasitemia* 24hrs | 48hrs | 72hrs | 96hrs | 120hrs |
|---|---|---|---|---|---|
| Control (no additions) | 3.8 | 7.1 | 10.8 | 18.0 | 30.0 |
| + 5mM ETR | 0.3 | 0.9 | 0.8 | 0.6 | 0.6 |
| + 2mM ETR | 0.5 | 0.6 | 0.8 | 0.7 | 0.5 |
| + 1mM ETR | 0.9 | 1.3 | 0.8 | 0.8 | 0.5 |

*The results reflect the average of counts from three independent investigators. The drug was removed after 48 hrs. and replaced by complete medium. That the parasitemia did not reappear after the drug was removed suggests that ETR is cidal.

Microscopic examination of Giemsa-stained smears showed that 5-ethylthioribose (ETR) was inhibitory to P. falciparium-infected cultures. The control culture produced a continuous and progressive infection. The anti-parasitic effect of ETR was apparent at the lowest concentration tested. After 24 hours in the presence of 1 mM ETR a small increase in growth was noted but the parasites appeared abnormal. By 48 hours the

parasitemia had decreased to less than 0.1% (initial seeding was <1.0%) with a noticeable amount of debris in the stained smears. By 72 hours the infection was assessed to be less than 1 parasite per 10,000 red cells and only abnormal pyknotic forms remained. 1mM and 5mM produced an immediate and pronounced effect on the course of the culture infection. In both cultures after 24 hours the parasitemia had remained at or below the level of the initial inoculum and only abnormal "crisis" forms could be seen. By 72 hours, all drug-treated cultures were completely negative. It is of interest that after 5 days (the last 3 days without drug) the parasitemia did not reappear.

The results show that ETR is inhibitory toward P. falciparum in vitro. In addition, all observers noted that only abnormal pyknotic forms existed in drug-treated cultures beyond 48 hours. These forms are called "crisis" forms and are considered to be non-viable parasites.

The preceding examples can be repeated with similar success by substituting the generically or specifically described reactants and/or operating conditions of this invention for those used in the preceding examples.

## Claims

**Claims for the following Contracting States : BE, FR, DE, IT, LU, NL, SE, CH, LI, GB**

**1.** A compound characterized by the formula:

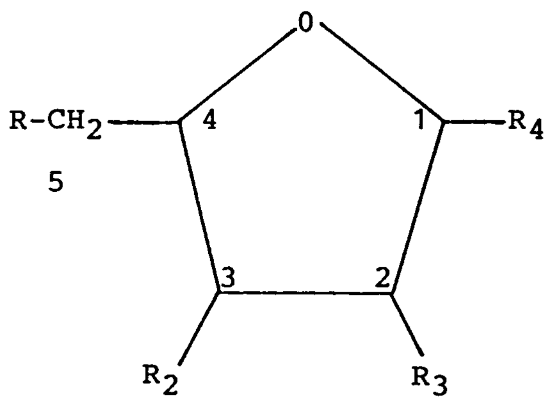

(Ia)

wherein R is $R_1S$- in which $R_1$ is a $C_1$-$C_{10}$ linear or branched chain alkyl or halogenated linear or branched chain alkyl,
wherein $R_2$, $R_3$ and $R_4$ are the same or different and each is H or OH,
with the proviso that at least one of $R_2$, $R_3$ and $R_4$ is hydroxy and the further proviso that when $R_2$, $R_3$ and $R_4$ are all OH, $R_1$ is halogenated linear or branched chain alkyl.

**2.** A compound of the formula Ib:

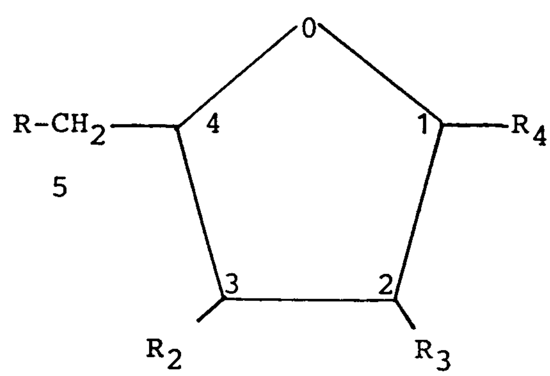

(Ib)

wherein R is H-, Cl-, F-, Br-, I- or $R_1S$-, in which $R_1$ is $C_1$-$C_{10}$ linear or branched chain alkyl or halogenated $C_1$-$C_{10}$ linear or branched chain alkyl,
wherein $R_2$, $R_3$ and $R_4$ are the same or different and each is H- or -OH with the proviso that at least one of $R_2$, $R_3$ and $R_4$ is -OH and the further proviso that when $R_2$, $R_3$ and $R_4$ are all -OH, $R_1$ is other

than methyl,
for use in human or veterinary medicine.

3. 5-chlororibose or 5-ethylthioribose for use in human or veterinary medicine.

4. A compound as claimed in claim 2 or 3 for use as an antifungal or antiprotozoal.

5. The use of compound of formula Ib:

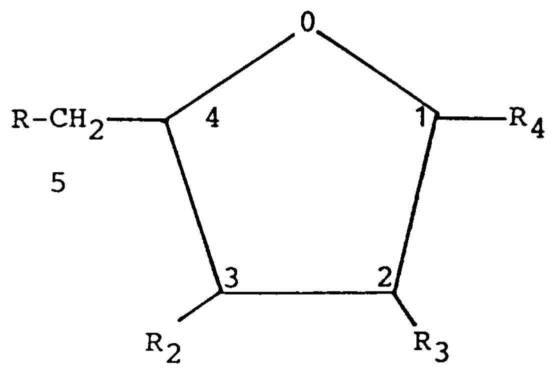

(Ib)

wherein R is H-, Cl-, F-, Br-, I- or $R_1S$-, in which $R_1$ is $C_1$-$C_{10}$ linear or branched chain alkyl or halogenated alkyl,
wherein $R_2$, $R_3$ and $R_4$ are the same or different and each is H- or -OH,
with the proviso that at least one of $R_2$, $R_3$ and $R_4$ is -OH and the further proviso that when $R_2$, $R_3$ and $R_4$ are all -OH, $R_1$ is other than methyl in the preparation of an antimicrobial agent.

6. The use as claimed in claim 5, wherein the antimicrobial agent is an antifungal agent or an antiprotozoal agent.

7. A pharmaceutical or veterinary composition comprising an amount of a compound effective to inhibit the growth of an MTR kinase-dependent microorganism, in a suitable carrier, wherein the compound is of formula Ib

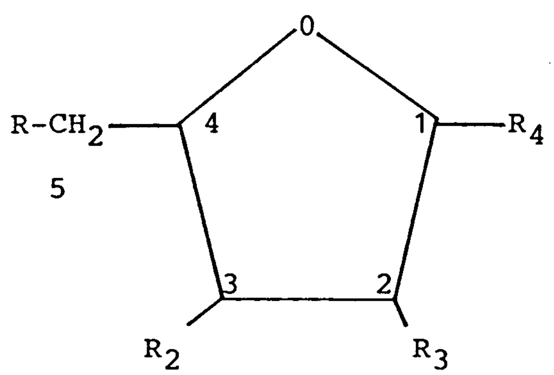

(Ib)

wherein R is H-, Cl-, F-, Br-, I- or $R_1S$-, in which $R_1$ is $C_1$-$C_{10}$ linear or branched chain alkyl or halogenated alkyl,
wherein $R_2$, $R_3$ and $R_4$ are the same or different and each is H- or -OH, with the proviso that at least one of $R_2$, $R_3$ and $R_4$ is -OH and the further proviso that when $R_2$, $R_3$ and $R_4$ are all -OH, $R_1$ is other than methyl.

8. A pharmaceutical or veterinary composition comprising 5-chlororibose or 5-ethylthioribose and a suitable carrier.

**9.** A process for the preparation of a compound of formula Ia

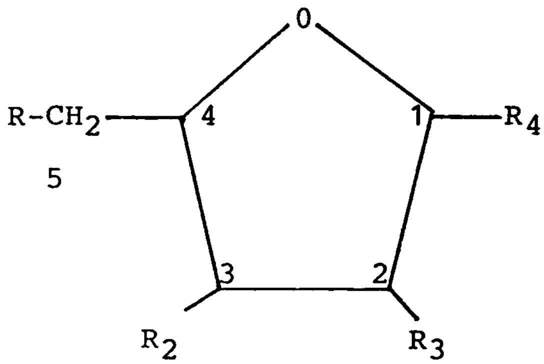

(Ia)

wherein R is $R_1S$- in which $R_1$ is a $C_1$-$C_{10}$ linear or branched chain alkyl or halogenated linear or branched alkyl,
wherein $R_2$, $R_3$ and $R_4$ are the same or different and each is H or OH,
with the proviso that at least one of $R_2$, $R_3$ and $R_4$ is hydroxy and the further proviso that when $R_2$, $R_3$ and $R_4$ are all OH, $R_1$ is halogenated linear or branched chain alkyl;
the method comprising hydrolysing a compound of the formula IIa

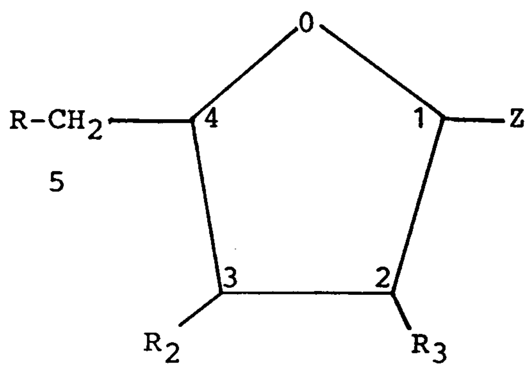

IIa

wherein R, $R_2$ and $R_3$ are defined above for formula Ia and Z is hydrolysable to a group $R_4$.

**Claims for the following Contracting States : AT, ES, GR**

**1.** A process for the preparation of a compound of formula Ia

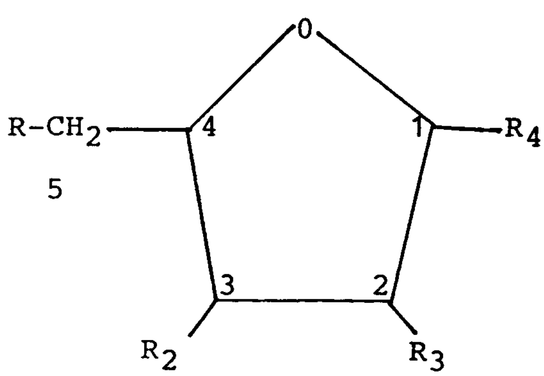

(Ia)

wherein R is $R_1S$- in which $R_1$ is a $C_1$-$C_{10}$ linear or branched chain alkyl or halogenated linear or branched alkyl,
wherein $R_2$, $R_3$ and $R_4$ are the same or different and each is H or OH,

with the proviso that at least one of $R_2$, $R_3$ and $R_4$ is hydroxy and the further proviso that when $R_2$, $R_3$ and $R_4$ are all OH, $R_1$ is halogenated linear or branched chain alkyl;
the method comprising hydrolysing a compound of the formula IIa

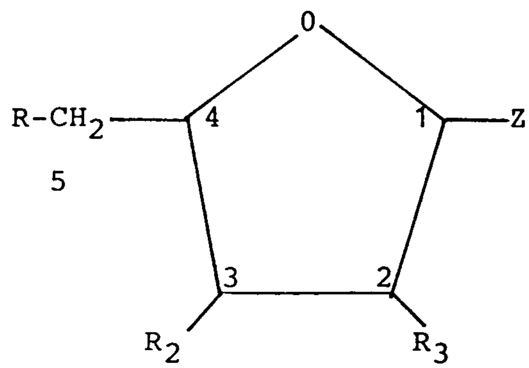

IIa

wherein R, $R_2$ and $R_3$ are defined above for formula Ia and Z is hydrolysable to a group $R_4$.

**2.** A process for preparing a pharmaceutical or veterinary composition, the process comprising admixing a compound of formula Ib:

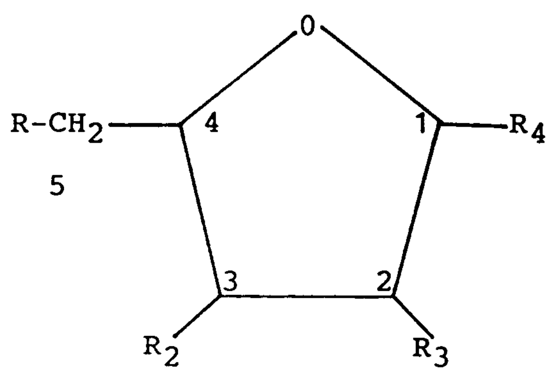

(Ib)

wherein R is H, Cl, Br, F, I or $R_1S$- in which $R_1$ is a $C_1$-$C_{10}$ linear or branched chain alkyl or halogenated linear or branched alkyl,
wherein $R_2$, $R_3$ and $R_4$ are the same or different and each is H or OH, with the proviso that at least one of $R_2$, $R_3$ and $R_4$ is OH and the further proviso that when $R_2$, $R_3$ and $R_4$ are all OH, $R_1$ is other than methyl;
with a pharmaceutically or veterinarily acceptable carrier.

**3.** A process for preparing a pharmaceutical or veterinary composition, the process comprising admixing 5-chlororibose or 5-ethylthioribose and a pharmaceutically or veterinarily acceptable carrier.

4. The use of compound of formula Ib:

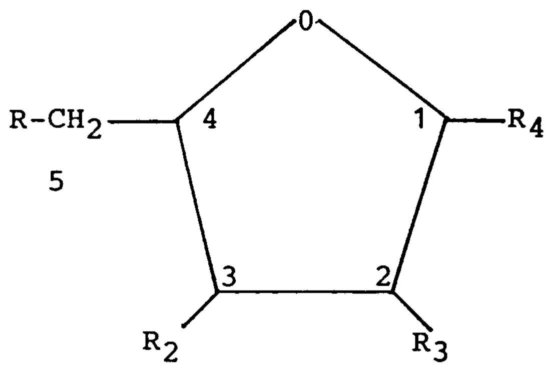

(Ib)

wherein R is H-, Cl-, F-, Br-, I- or $R_1S$-, in which $R_1$ is $C_1$-$C_{10}$ linear or branched chain alkyl or halogenated alkyl,
wherein $R_2$, $R_3$ and $R_4$ are the same or different and each is H- or -OH,
with the proviso that at least one of $R_2$, $R_3$ and $R_4$ is -OH and the further proviso that when $R_2$, $R_3$ and $R_4$ are all -OH, $R_1$ is other than methyl in the preparation of an antimicrobial agent.

5. The use as claimed in claim 4, wherein the antimicrobial agent is an antifungal agent or an antiprotozoal agent.

6. A process as claimed in claim 1 or 2, wherein $R_2$, $R_3$ and $R_4$ are all OH.

7. A process as claimed in claim 2, wherein R is Cl.

8. A process as claimed in claim 2, wherein R is $R_1S$-.

9. A process as claimed in claim 8, wherein $R_1$ is $CH_3CH_2$-.

**Patentansprüche**
**Patentansprüche für folgende Vertragsstaaten : BE, FR, DE, IT, LU, NL, SE, CH, LI, GB**

1. Verbindung, gekennzeichnet durch die Formel:

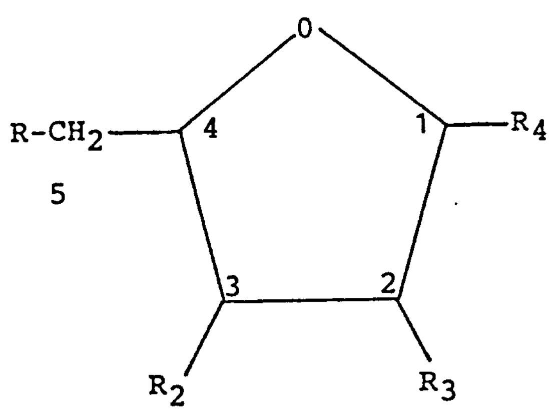

(Ia)

worin R $R_1S$- ist, in welchem $R_1$ ein $C_1$-$C_{10}$ lineares oder verzweigtes Kettenalkyl oder ein halogeniertes, lineares oder verzweigtes Kettenalkyl ist,
worin $R_2$, $R_3$ und $R_4$ gleich oder verschieden sind und jedes H oder OH ist,
unter der Voraussetzung, daß wenigstens eines von $R_2$, $R_3$ und $R_4$ Hydroxy ist, und unter der weiteren Voraussetzung, daß, wenn $R_2$, $R_3$ und $R_4$ alle OH sind, $R_1$ ein halogeniertes, lineares oder verzweigtes Kettenalkyl ist.

**2.** Verbindung mit der Formel Ib:

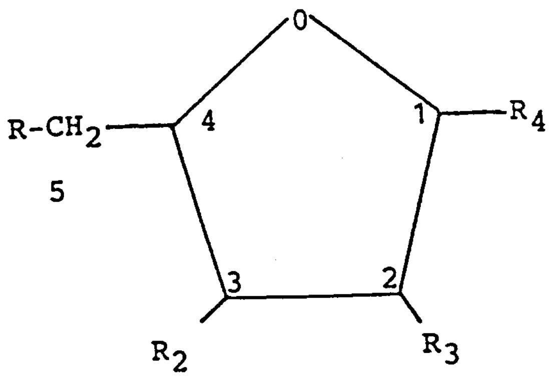

(Ib)

worin R H-, Cl-, F-, Br-, I- oder $R_1S$- ist, in welchem $R_1$ ein $C_1$-$C_{10}$ lineares oder verzweigtes Kettenalkyl oder halogeniertes $C_1$-$C_{10}$ lineares oder verzweigtes Kettenalkyl ist,
worin $R_2$, $R_3$ und $R_4$ gleich oder verschieden sind und jedes H- oder -OH ist, unter der Voraussetzung, daß mindestens eines von $R_2$, $R_3$ und $R_4$ -OH ist, und unter der weiteren Voraussetzung, daß, wenn $R_2$, $R_3$ und $R_4$ -OH sind, $R_1$ etwas anderes als Methyl ist,
zur Verwendung in der Human- oder Veterinärmedizin.

**3.** 5-Chlororibose oder 5-Ethylthioribose zur Verwendung in der Human- oder Veterinärmedizin.

**4.** Verbindung nach Anspruch 2 oder 3 zur Verwendung als Antimykotikum oder Antiprotozoikum.

**5.** Verwendung einer Verbindung mit der Formel Ib:

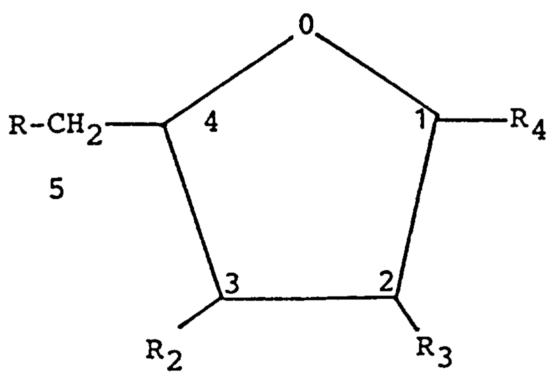

(Ib)

worin R H-, Cl-, F-, Br-, I- oder $R_1S$- ist, in welchem $R_1$ ein $C_1$-$C_{10}$ lineares oder verzweigtes Kettenalkyl oder halogeniertes Alkyl ist,
worin $R_2$, $R_3$ und $R_4$ gleich oder verschieden sind und jedes H- oder -OH ist,
unter der Voraussetzung, daß zumindest eines von $R_2$, $R_3$ und $R_4$ -OH ist, und unter der weiteren Voraussetzung, daß, wenn $R_2$, $R_3$ und $R_4$ alle -OH sind, $R_1$ etwas anderes als Methyl ist, bei der Herstellung eines antimikrobiellen Wirkstoffes.

**6.** Verwendung nach Anspruch 5, worin der antimikrobielle Wirkstoff ein Antimykotikum oder ein Antiprotozoikum ist.

**7.** Pharmazeutisehe oder veterinäre Zusammensetzung, welche eine Verbindung in einer Menge, die wirksam ist, das Wachstum eines MTR-Kinaseabhängigen Mikroorganismus zu bremsen, in einem geeigneten Träger enthält, wobei die Verbindung die Formel Ib aufweist,

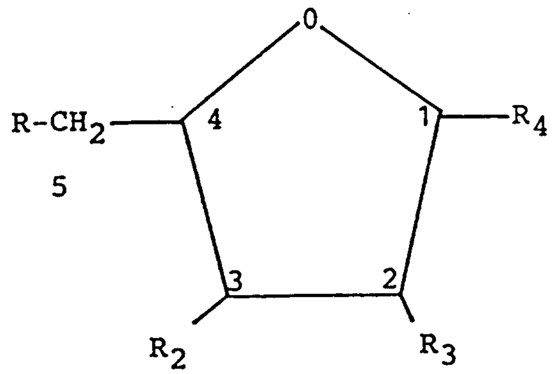

(Ib)

worin R H-, Cl-, F-, Br-, I- oder $R_1S$- ist, in welchem $R_1$ ein $C_1$-$C_{10}$ lineares oder verzweigtes Kettenalkyl oder halogeniertes Alkyl ist,
worin $R_2$, $R_3$ und $R_4$ gleich oder verschieden sind und jedes H- oder -OH ist, unter der Voraussetzung, daß mindestens eines von $R_2$, $R_3$ und $R_4$ -OH ist, und unter der weiteren Voraussetzung, daß, wenn $R_2$, $R_3$ und $R_4$ -OH sind, $R_1$ etwas anderes als Methyl ist.

**8.** Pharmazeutische oder veterinäre Zusammensetzung, welche 5-Chlororibose oder 5-Ethylthioribose und einen geeigneten Träger enthält.

**9.** Verfahren zur Herstellung einer Verbindung mit der Formel Ia,

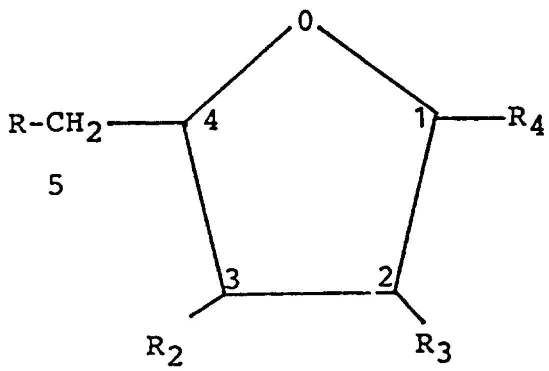

(Ia)

worin R $R_1S$- ist, in welchem $R_1$ ein $C_1$-$C_{10}$ lineares oder verzweigtes Kettenalkyl oder ein halogeniertes, lineares oder verzweigtes Kettenalkyl ist,
worin $R_2$, $R_3$ und $R_4$ gleich oder verschieden sind und jedes H oder OH ist,
unter der Voraussetzung, daß wenigstens eines von $R_2$, $R_3$ und $R_4$ Hydroxy ist, und unter der weiteren Voraussetzung, daß, wenn $R_2$, $R_3$ und $R_4$ alle OH sind, $R_1$ ein halogeniertes, lineares oder verzweigtes Kettenalkyl ist,
wobei das Verfahren die Hydrolyse einer Verbindung mit der Formel IIa

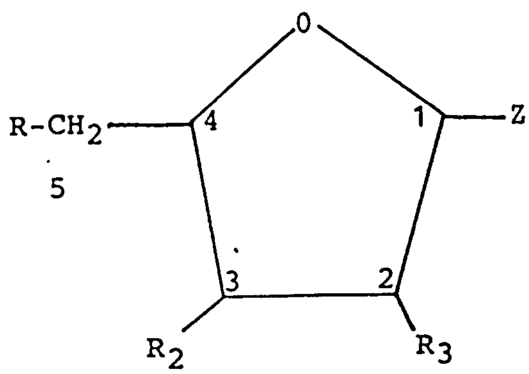

IIa

beinhaltet,
worin R, $R_2$ und $R_3$ oben für die Formel Ia definiert sind, und Z zu einer Gruppe $R_4$ hydrolysierbar ist.

**Patentansprüche für folgende Vertragsstaaten : AT, ES, GR**

**1.** Verfahren zur Herstellung einer Verbindung mit der Formel Ia

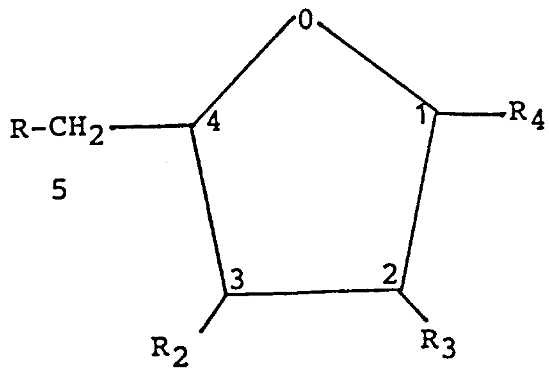

(Ia)

worin R $R_1$S- ist, in welchem $R_1$ ein $C_1$-$C_{10}$ lineares oder verzweigtes Kettenalkyl oder ein halogeniertes, lineares oder verzweigtes Alkyl ist,
worin $R_2$, $R_3$ und $R_4$ gleich oder verschieden sind und jedes H oder OH ist,
unter der Voraussetzung, daß wenigstens eines von $R_2$, $R_3$ und $R_4$ Hydroxy ist, und unter der weiteren Voraussetzung, daß, wenn $R_2$, $R_3$ und $R_4$ alle OH sind, $R_1$ ein halogeniertes, lineares oder verzweigtes Kettenalkyl ist,
wobei das Verfahren das Hydrolysieren einer Verbindung mit der Formel IIa

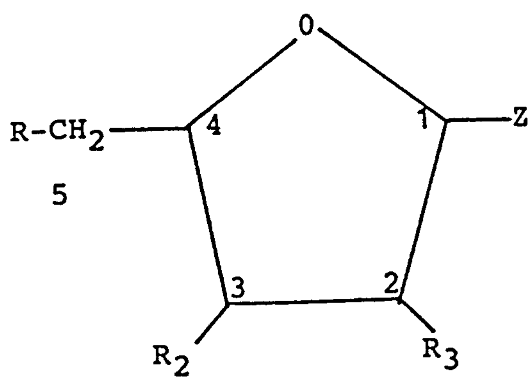

IIa

beinhaltet,
worin R, $R_2$ und $R_3$ oben für die Formel Ia definiert sind, und Z zu einer Gruppe $R_4$ hydrolysierbar ist.

**2.** Verfahren zur Herstellung einer pharmazeutischen oder veterinären Zusammensetzung, wobei das Verfahren das Zumischen einer Verbindung mit der Formel Ib:

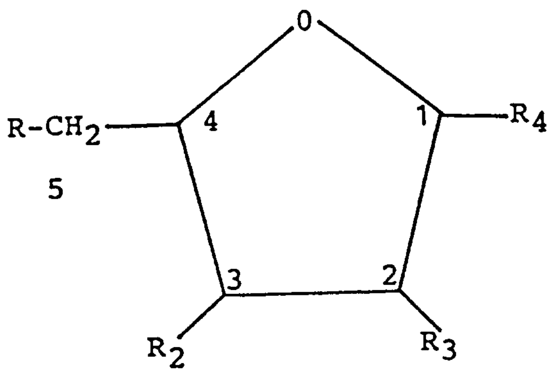

(Ib)

worin R H, Cl, Br, F, I oder $R_1S$- ist, in welchem $R_1$ ein $C_1$-$C_{10}$ lineares oder verzweigtes Kettenalkyl oder halogeniertes $C_1$-$C_{10}$ lineares oder verzweigtes Alkyl ist,
worin $R_2$, $R_3$ und $R_4$ gleich oder verschieden und jedes H- oder -OH ist, unter der Voraussetzung, daß mindestens eines von $R_2$, $R_3$ und $R_4$ -OH ist, und unter der weiteren Voraussetzung, daß, wenn $R_2$, $R_3$ und $R_4$ alle -OH sind, $R_1$ etwas anderes als Methyl ist,
zu einem pharmazeutisch oder veterinär verträglichen Träger beinhaltet.

**3.** Verfahren zur Herstellung einer pharmazeutisch oder veterinären Zusammensetzung, wobei das Verfahren ein Zumischen von 5-Chlororibose oder 5-Ethylthioribose zu einem pharmazeutisch oder veterinär verträglichen Träger beinhaltet.

**4.** Verwendung einer Verbindung mit der Formel Ib:

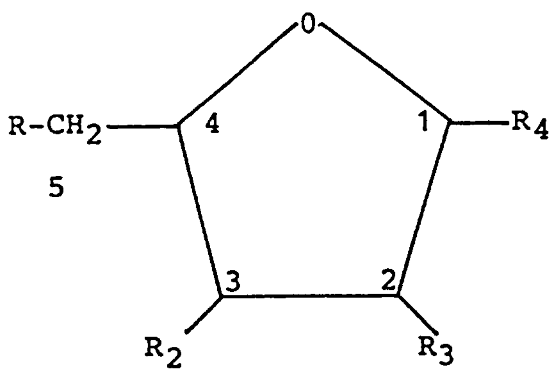

(Ib)

worin R H-, Cl-, F-, Br-, I- oder $R_1S$- ist, in welchem $R_1$ ein $C_1$-$C_{10}$ lineares oder verzweigtes Kettenalkyl oder hälogeniertes Alkyl ist,
worin $R_2$, $R_3$ und $R_4$ gleich oder verschieden sind und jedes H- oder -OH ist,
unter der Voraussetzung, daß mindestens eines von $R_2$, $R_3$ und $R_4$ -OH ist, und unter der weiteren Voraussetzung, daß, wenn $R_2$, $R_3$ und $R_4$ -OH sind, $R_1$ etwas anderes als Methyl ist,
bei der Herstellung eines aptimikrobiellen Wirkstoffes.

**5.** Verwendung nach Anspruch 4, worin das Bakteriostatikum ein Antimykotikum oder ein Antiprotozoikum ist.

**6.** Verfahren nach Anspruch 1 oder 2, worin $R_2$, $R_3$ und $R_4$ alle OH sind.

**7.** Verfahren nach Anspruch 2, worin R Cl ist.

**8.** Verfahren nach Anspruch 2, worin R $R_1S$- ist.

**9.** Verfahren nach Anspruch 8, worin $R_1$ $CH_3CH_2$- ist.

**Revendications**

**Revendications pour les Etats contractants suivants : BE, FR, DE, IT, LU, NL, SE, CH, LI, GB**

**1.** Composé de formule :

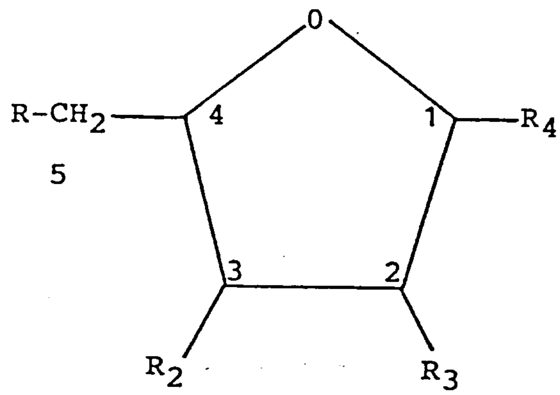

(Ia)

où R est $R_1S$- dans lequel $R_1$, est un alcoyle en chaîne linéaire ou ramifiée ou alcoyle en chaîne linéaire ou ramifiée halogéné en $C_{1-10}$,

où $R_2$, $R_3$ et $R_4$ sont identiques ou différents et chacun est H ou OH,

avec la restriction qu'au moins l'un d'entre $R_2$, $R_3$ et $R_4$ est hydroxyle et avec la restriction supplémentaire que lorsque $R_2$, $R_3$ et $R_4$ sont tous hydroxyle, $R_1$ est alcoyle en chaîne linéaire ou ramifiée halogéné.

**2.** Composé de formule Ib :

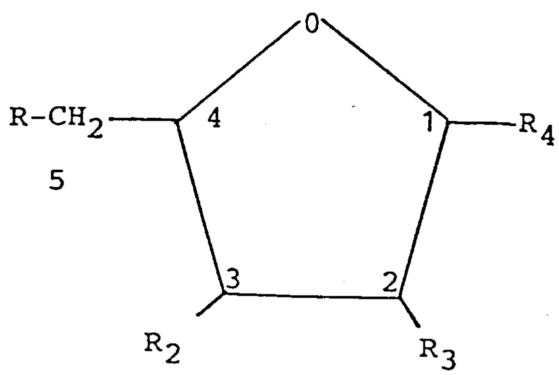

(Ib)

où R est H-, Cl-, F-, Br-, I- ou $R_1S$-, dans lequel $R_1$ est alcoyle en chaîne linéaire ou ramifiée en $C_{1-10}$ ou alcoyle en chaîne linéaire ou ramifiée en $C_{1-10}$ halogéné,

où $R_2$, $R_3$ et $R_4$ sont identiques ou différents et chacun est H- ou -OH,

avec la restriction qu'au moins l'un d'entre $R_2$, $R_3$ et $R_4$ est -OH et avec la restriction supplémentaire que lorsque $R_2$, $R_3$ et $R_4$ sont tous -OH, $R_1$ est autre que méthyle,

à utiliser en médecine humaine ou vétérinaire.

**3.** Le 5-chlororibose ou le 5-éthylthioribose à utiliser en médecine humaine ou vétérinaire.

**4.** Composé suivant la revendication 2 ou 3, à utiliser comme antifongique ou antiprotozoaire.

**5.** Utilisation d'un composé de formule Ib :

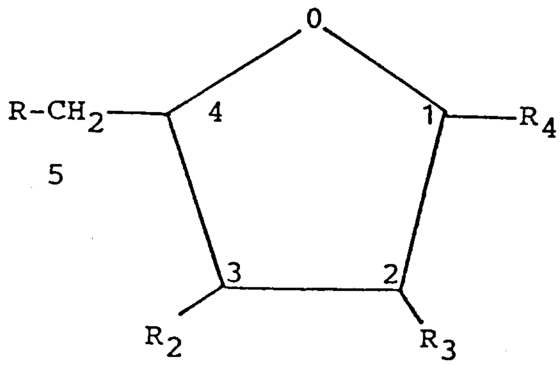

(Ib)

où R est H-, Cl-, F-, Br-, I- ou $R_1S$-, dans lequel $R_1$ est alcoyle ou alcoyle halogéné en chaîne linéaire ou ramifiée en $C_{1-10}$,

où $R_2$, $R_3$ et $R_4$ sont identiques ou différents et chacun est H- ou -OH,

avec la restriction qu'au moins l'un d'entre $R_2$, $R_3$ et $R_4$ est -OH et avec la restriction supplémentaire que lorsque $R_2$, $R_3$ et $R_4$ sont tous -OH, $R_1$ est autre que méthyle,

dans la préparation d'un agent antimicrobien.

**6.** Utilisation suivant la revendication 5, dans laquelle l'agent antimicrobien est un agent antifongique ou un agent antiprotozoaire.

**7.** Composition pharmaceutique ou vétérinaire, comprenant une quantité d'un composé efficace pour inhiber la croissance d'un micro-organisme dépendant de la MTR kinase, dans un excipient approprié, dans laquelle le composé est de formule Ib :

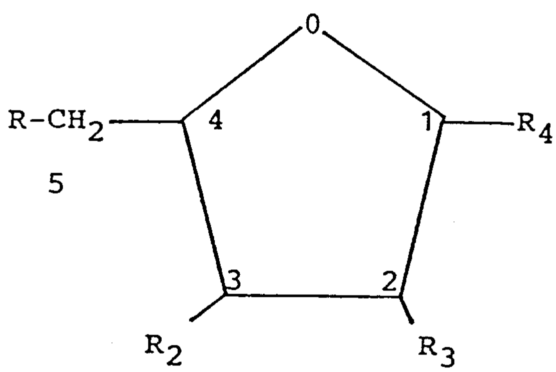

(Ib)

où R est H-, Cl-, F-, Br-, I- ou $R_1S$-, dans lequel $R_1$ est alcoyle ou alcoyle halogéné en chaîne linéaire ou ramifiée en $C_{1-10}$,

où $R_2$, $R_3$ et $R_4$ sont identiques ou différents et chacun est H- ou -OH,

avec la restriction qu'au moins l'un d'entre $R_2$, $R_3$ et $R_4$ est -OH et avec la restriction supplémentaire que lorsque $R_2$, $R_3$ et $R_4$ sont tous -OH, $R_1$ est autre que méthyle.

**8.** Composition pharmaceutique ou vétérinaire, comprenant du 5-chlororibose ou du 5-éthylthioribose et un excipient approprié.

**9.** Procédé de préparation d'un composé de formule Ia :

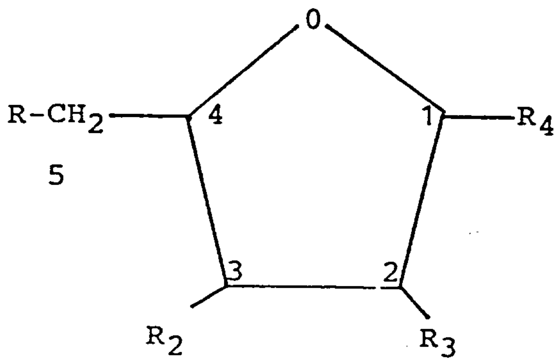

(Ia)

où R est $R_1S$-, dans lequel $R_1$ est un alcoyle en chaîne linéaire ou ramifiée ou alcoyle en chaîne linéaire ou ramifiée halogéné en $C_{1-10}$,

où $R_2$, $R_3$ et $R_4$ sont identiques ou différents et chacun est H ou OH,

avec la restriction qu'au moins l'un d'entre $R_2$, $R_3$ et $R_4$ est hydroxyle et avec la restriction supplémentaire que lorsque $R_2$, $R_3$ et $R_4$ sont tous hydroxyle, $R_1$ est alcoyle en chaîne linéaire ou ramifiée halogéné,

le procédé comprenant l'hydrolyse d'un composé de formule IIa :

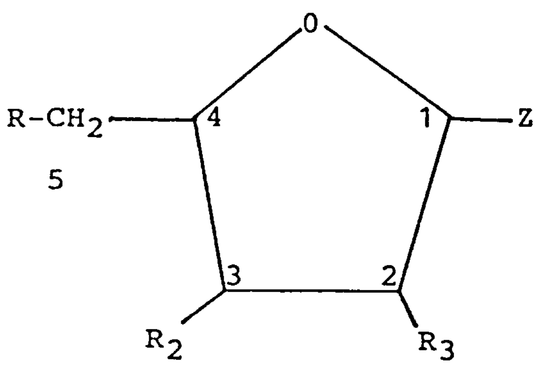

(IIa)

où R, $R_2$ et $R_3$ sont tels que définis à propos de la formule Ia et Z est hydrolysable en un radical $R_4$.

**Revendications pour les Etats contractants suivants : AT, ES, GR**

**1.** Procédé de préparation d'un composé de formule Ia :

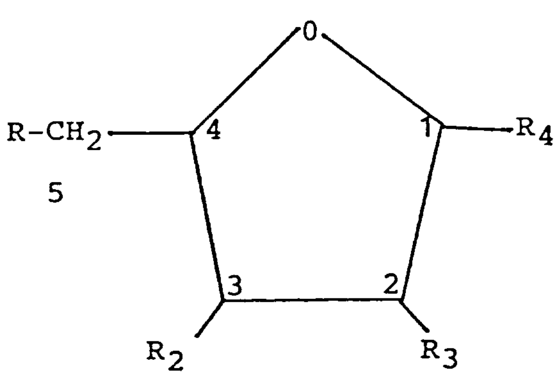

(Ia)

où R est $R_1S$-, dans lequel $R_1$ est un alcoyle en chaîne linéaire ou ramifiée ou alcoyle en chaîne linéaire ou ramifiée halogéné en $C_{1-10}$,

où $R_2$, $R_3$ et $R_4$ sont identiques ou différents et chacun est H ou OH,
avec la restriction qu'au moins l'un d'entre $R_2$, $R_3$ et $R_4$ est hydroxyle et avec la restriction supplémentaire que lorsque $R_2$, $R_3$ et $R_4$ sont tous hydroxyle, $R_1$ est alcoyle en chaîne linéaire ou ramifiée halogéné,
le procédé comprenant l'hydrolyse d'un composé de formule IIa :

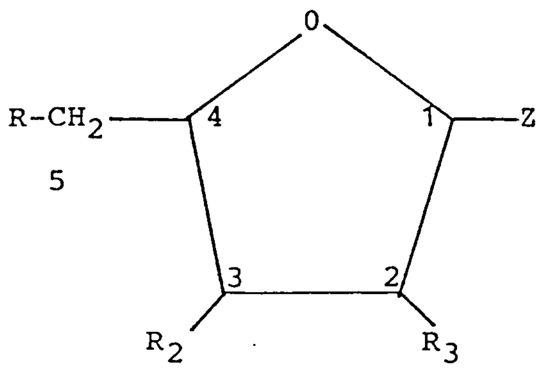

(IIa)

où R, $R_2$ et $R_3$ sont tels que définis à propos de la formule Ia et Z est hydrolysable en un radical $R_4$.

**2.** Procédé de préparation d'une composition pharmaceutique ou vétérinaire, le procédé comprenant le mélange d'un composé de formule Ib :

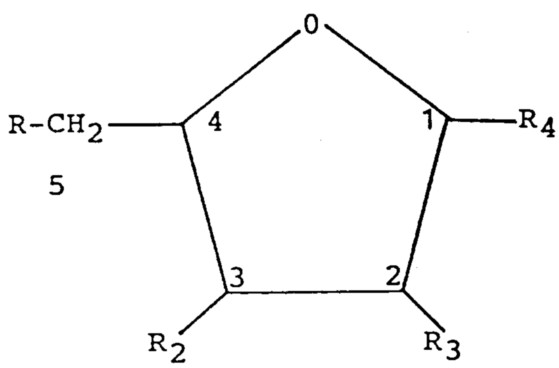

(Ib)

où R est H-, Cl-, F-, Br-, I- ou $R_1$S-, dans lequel $R_1$ est alcoyle en chaîne linéaire ou ramifiée ou alcoyle en chaîne linéaire ou ramifiée halogéné en $C_{1-10}$,
où $R_2$, $R_3$ et $R_4$ sont identiques ou différents et chacun est H- ou -OH,
avec la restriction qu'au moins l'un d'entre $R_2$, $R_3$ et $R_4$ est -OH et avec la restriction supplémentaire que lorsque $R_2$, $R_3$ et $R_4$ sont tous -OH, $R_1$ est autre que méthyle,
avec un excipient pharmaceutiquement ou vétérinairement acceptable.

**3.** Procédé de préparation d'une composition pharmaceutique ou vétérinaire, le procédé comprenant le mélange du 5-chlororibose ou du 5-éthylthioribose avec un excipient pharmaceutiquement ou vétérinairement acceptable.

**4.** Utilisation d'un composé de formule Ib :

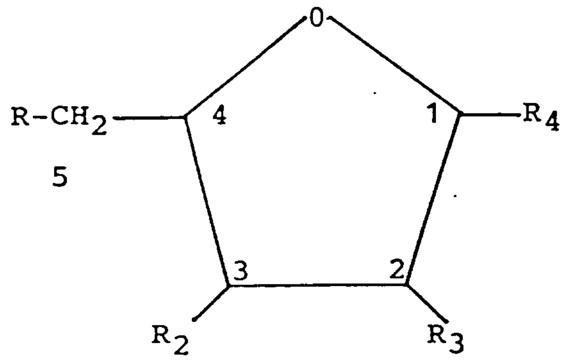

(Ib)

où R est H-, Cl-, F-, Br-, I- ou $R_1S$-, dans lequel $R_1$ est alcoyle ou alcoyle halogéné en chaîne linéaire ou ramifiée en $C_{1-10}$,

où $R_2$, $R_3$ et $R_4$ sont identiques ou différents et chacun est H- ou -OH,

avec la restriction qu'au moins l'un d'entre $R_2$, $R_3$ et $R_4$ est -OH et avec la restriction supplémentaire que lorsque $R_2$, $R_3$ et $R_4$ sont tous -OH, $R_1$ est autre que méthyle,

dans la préparation d'un agent antimicrobien.

**5.** Utilisation suivant la revendication 4, dans laquelle l'agent antimicrobien est un agent antifongique ou un agent antiprotozoaire.

**6.** Procédé suivant la revendication 1 ou 2, dans lequel $R_2$, $R_3$ et $R_4$ sont tous OH.

**7.** Procédé suivant la revendication 2, dans lequel R est Cl.

**8.** Procédé suivant la revendication 2, dans lequel R est $R_1S$-.

**9.** Procédé suivant la revendication 8, dans lequel $R_1$ est $CH_3CH_2$.